Europäisches Patentamt

European Patent Office

Office européen des brevets

(11) Numéro de publication: **0 271 374 B1**

(12) **FASCICULE DE BREVET EUROPEEN**

(45) Date de publication de fascicule du brevet:
**29.01.92**

(51) Int. Cl.⁵: **A61K 31/65, A61K 47/22**

(21) Numéro de dépôt: **87402498.7**

(22) Date de dépôt: **05.11.87**

Le dossier contient des informations techniques présentées postérieurement au dépôt de la demande et ne figurant pas dans le présent fascicule.

(54) **Composition antibiotique aqueuse à usage vétérinaire.**

(30) Priorité: **10.12.86 FR 8617270**

(43) Date de publication de la demande:
**15.06.88 Bulletin 88/24**

(45) Mention de la délivrance du brevet:
**29.01.92 Bulletin 92/05**

(84) Etats contractants désignés:
**AT BE CH DE ES GB GR IT LI LU NL SE**

(56) Documents cités:
**EP-A- 0 125 725**
**EP-A- 0 129 285**
**DE-B- 1 053 736**
**US-A- 4 081 528**
**US-A- 4 086 332**

(73) Titulaire: **SO.GE.VAL S.A.**
**200 Route de Mayenne**
**Laval Mayenne(FR)**

(72) Inventeur: **Daoudal, José**
**10 Avenue Général Patton**
**Laval Mayenne(FR)**

(74) Mandataire: **Cabinet Pierre HERRBURGER**
**115, Boulevard Haussmann**
**F-75008 Paris(FR)**

# Description

La présente invention concerne une composition antibiotique aqueuse à usage vétérinaire.

Cette composition est plus particulièrement destinée à des mammifères : bovins, ovins, caprins, porcins, équins, lapins ou même des volailles.

Il s'agit là d'un produit plus spécialement destiné à être injecté par voie intramusculaire.

La voie intraveineuse ou sous cutanée pourrait également être utilisée en adaptant la concentration en principe actif.

Les vétérinaires sont souvent confrontés aux problèmes liés à la nécessité d'injecter aux animaux une quantité d'antibiotique importante sous un faible volume et d'assurer une couverture antibiotique prolongée.

Dans ce but, diverses compositions ont déjà été proposées, mais celles-ci ne sont que peu satisfaisantes, étant donné que les antibiotiques usuels sont généralement très difficiles à dissoudre en quantités importantes en terme d'activité dans des solutions aqueuses. Différents additifs ont déjà été proposés pour améliorer cette solubilité, mais, ceux-ci présentent souvent des inconvénients du point de vue pharmacologique et leur innocuité n'est pas toujours établie.

La présente invention a pour objet de remédier à ces inconvénients en proposant une composition antibiotique aqueuse à usage vétérinaire susceptible d'être injectée par voie intramusculaire sous une forme très concentrée et ce, sans risquer d'entraîner des effets secondaires défavorables.

Cette composition s'est avérée efficace pour le traitement de nombreuses affections, notamment et préférentiellement des infections respiratoires.

Selon l'invention, cette composition est caractérisée en ce qu'elle renferme, en tant que principe actif, une tétracycline connue, notamment sous forme de chlorhydrate, dissoute dans la N(hydroxy éthyl)-2-pyrrolidone en milieu faiblement acide à neutre et en présence d'un composé de magnésium, notamment d'oxyde de magnésium.

On a, en effet, pu constater, de manière surprenante, que les tétracyclines forment, avec certains composés du magnésium, notamment les oxydes, des complexes qui sont solubles dans des solution aqueuses de N(hydroxy éthyl)-2-pyrrolidone à un pH environ compris entre 5 et 7,5.

Il s'agit là d'une propriété particulièrement avantageuse des tétracyclines, étant donné que la N(hydroxy éthyl)-2-pyrrolidone ne présente aucune toxicité pour l'organisme animal.

On avait déjà proposé, notamment conformément au document US-A-4 086 332, des compositions antibiotiques renfermant en tant que solvant de la 2-pyrrolidone et non son dérivé substitué N-(hydroxy éthyl)-2-pyrrolidone.

Le document EP-A-0 129 285 décrit bien quant à lui des préparations pharmaceutiques renfermant divers principes actifs, notamment des tétracyclines et le dérivé substitué N(2-hydroxy éthyl)-pyrrolidone. Le rôle de ce dernier composant est toutefois d'améliorer la pénétration dans la peau de ces préparations en agissant conjointement avec un agent troublant l'enveloppe cellulaire pouvant être par exemple constitué par de l'acide oléique. Il n'est nullement fait allusion dans ce document à une quelconque amélioration de la solubilité dans l'eau du principe actif et ce d'autant plus qu'il est même indiqué expressément que l'élément améliorant la pénétration dans la peau peut ou non se trouver dans la composition à l'état dissous.

La composition selon l'invention peut, bien entendu, trouver son utilité pour le traitement de toutes les affections pour lesquelles on a l'habitude d'avoir recours aux tétracyclines.

Parmi les tétracyclines pouvant être présentes dans la composition conforme à l'invention, on peut mentionner l'oxytétracycline, la tétracycline, la chlortétracycline et plus particulièrement la doxycycline.

Cette composition est destinée à être appliquée avec le dosage usuel, correspondant à 10 mg de principe actif (doxycycline base) par kg de poids vif et par jour.

Selon une autre caractéristique de l'invention, la composition comprend entre 5 et 40 % en poids de chlorhydrate de doxycycline ayant une activité au moins égale à 822 $U^I$/mg, c'est-à-dire correspondant au chlorhydrate de doxycycline généralement accessible dans le commerce.

Les solutions utilisées préférentiellement, conformément à l'invention, contiennent, de préférence, de l'ordre de 20 g de doxycycline pour 100 cl de solution : il s'agit donc là de solutions relativement concentrées.

Selon une autre caractéristique de l'invention, la composition contient entre 5 et 80 % en poids de 2-hydroxy-éthyl-pyrrolidone, de préférence de l'ordre de 50 % en poids.

Par ailleurs, pour obtenir le pH acide compris entre 5 et 7,5 permettant la dissolution de la tétracycline dans la 2-hydroxy-éthyl-pyrrolidone en présence d'un composé du magnésium, il est nécessaire d'ajouter un acide à la composition, notamment de l'acide chlorhydrique concentré.

Selon une autre caractéristique de l'invention, la composition comporte du formaldéhyde sulfoxylate de sodium qui joue le rôle d'agent de conservation.

L'invention se rapporte également un procédé d'obtention de la composition décrite ci-dessus.

Ce procédé est caractérisé en ce que l'on mélange approximativement deux parties en poids de 2-hydroxy-éthyl-pyrrolidone pour une partie en poids d'eau distillée, on porte la température à environ 50°C, et, sous agitation, on ajoute un composé, notamment de l'oxyde de magnésium, puis, progressivement une tétracycline ou l'un de ses sels, notamment le chlorhydrate de doxycycline, on porte alors le pH à une valeur environ comprise entre 5 et 7,5 par addition d'acide chlorhydrique concentré et on complète avec de l'eau distillé/

On a vérifié qu'il est également possible d'ajouter l'acide chlorhydrique directement, avant la doxycycline, mais ce mode opératoire présente l'inconvénient d'obliger à connaître préalablement la dose d'acide devant être ajoutée.

Les caractéristiques de la composition qui fait l'objet de l'invention seront mieux comprises à la lecture de l'exemple suivant qui décrit les opérations nécessaires à la préparation de 100 ml d'une composition conforme à l'invention :

Pour obtenir 100 ml de solution, on pèse tout d'abord 50 g de 2-hydroxy-éthyl-pyrrolidone, on ajoute 25 g d'eau distillée et on porte la température à 50°C. Sous agitation, on ajoute ensuite 1 g de formaldéhyde sulfoxylate de sodium qui joue le rôle de conservateur puis 3,80 g d'oxyde de magnésium.

Ensuite et toujours sous agitation, on ajoute progressivement 20 g de doxycycline (sous forme de chlorhydrate) ; la solution obtenue, d'abord jaune foncé, s'éclaircit jusqu'à une teinte jaune laiteuse, correspondant à la formation du complexe de magnésium.

On ajoute, ensuite, progressivement de l'acide chlorhydrique concentré jusqu'à solubilisation et stabilisation du pH. Il ne reste alors plus qu'à compléter à 100 ml à l'aide d'eau distillée.

## Revendications

1. Composition antibiotique aqueuse à usage vétérinaire, caractérisée en ce qu'elle renferme en tant que principe actif, une tétracycline connue, notamment sous forme de chlorhydrate, dissoute dans la N(hydroxy éthyl)-2-pyrrolidone en milieu faiblement acide à neutre et en présence d'un composé de magnésium, notamment d'oxyde de magnésium.

2. Composition selon la revendication 1, caractérisée en ce que la tétracycline est choisie dans le groupe formé pas l'oxytétracycline, la doxycycline, la tétracycline et la chlortétracycline.

3. Composition selon la revendication 2, caractérisée en ce que la tétracycline est la doxycycline.

4. Composition selon l'une quelconque des revendications 1 à 3, caractérisé en ce qu'elle contient de l'acide chlorhydrique.

5. Composition selon l'une quelconque des revendications 1 à 4, caractérisée en ce qu'elle comporte du formaldéhyde sulfoxylate de sodium qui joue le rôle d'agent de conservation.

6. Composition selon l'une quelconque des revendications 1 à 5, caractérisée en ce qu'elle contient entre 5 et 40 % en poids de doxycline sous forme de chlorhydrate ayant une activité au moins égale à 822 UI/mg.

7. Composition selon l'une quelconque des revendications 1 à 6, caractérisée en ce qu'elle contient entre 5 et 80 % en poids de N-(hydroxy éthyl)-2-pyrrolidone.

8. Composition selon l'une quelconque des revendications 1 à 7, caractérisée en ce qu'elle est utilisée par voie injectable.

9. Procédé d'obtention d'une composition selon l'une quelconque des revendications 1 à 8, caractérisé en ce que l'on mélange deux parties en poids de N(hydroxy éthyl)-2-pyrrolidone pour une partie en poids d'eau distillée, on porte la température à environ 50°C, et, sous agitation, on ajoute un composé, notamment de l'oxyde de magnésium, puis, progressivement la tétracycline, notamment le chlorhydrate de doxycycline, on porte alors le pH à une valeur environ comprise entre 5 et 7,5, par addition d'acide chlorhydrique concentré et on complète avec de l'eau distillée.

## Claims

1. Aqueous antibiotic composition for veterinary use, characterised in that it contains as the active ingredient a known tetracycline, in particular in the form of hydrochloride, dissolved in N(hydroxyethyl)-2-pyrrolidone in a medium which is weakly acid to neutral and in the presence of a magnesium compound, in particular magnesium oxide.

2. Composition according to Claim 1, characterised in that the tetracycline is selected from the group comprising oxytetracycline, doxycycline, tetracycline and tetracycline chloride.

3. Composition according to Claim 2, characterised in that the tetracycline is doxycycline.

4. Composition according to any one of Claims 1 to 3, characterised in that it contains hydrochloric acid.

5. Composition according to any one of Claims 1 to 4, characterised in that it comprises sodium formaldehyde sulfoxylate which acts as a preserving agent.

6. Composition according to any one of Claims 1 to 5, characterised in that it contains between 5 to 40 weight % of doxycycline in the form of hydrochloride having an activity that it at least 822 UI/mg.

7. Composition according to any one of Claims 1 to 6, characterised in that it contains between 5 and 80 weight % of N(hydroxyethyl)-2-pyrrolidone.

8. Composition according to any one of Claims 1 to 7, characterised in that it is can be injected.

9. Process for obtaining a composition according to any one of Claims 1 to 8, characterised in that two parts by weight of N(hydroxyethyl)-2-pyrrolidine per one part by weight of distilled water are mixed, the temperature being brought to approximately 50° C, and, while the mixture is being stirred, a compound, in particular magnesium oxide, is added and then, progressively, tetracycline in particular doxycycline hydrochloride is added, the pH then brought to a value between 5 and 7.5 by the addition of concentrated hydrochloric acid, and the rpcess is completed with distilled water.

**Patentansprüche**

1. Wässerige antibiotische Zusammensetzung zu veterinärem Gebrauch,
**dadurch gekennzeichnet,**
daß die Zusammensetzung als wirksamen Grundbestandteil ein bereits bekanntes Tetracyclin enthält, hauptsächlich in Form von Chlorhydrat, aufgelöst in N(Hydroxyethyl)-2-Pyrrolidon in schwach saurem bis neutralem Milieu in Gegenwart einer Magnesiumverbindung, insbesondere von Magnesiumoxid.

2. Zusammensetzung nach Anspruch 1,
**dadurch gekennzeichnet,**
daß das Tetracyclin aus der Gruppe ausgewählt wird, die aus Oxytetracyclin, Doxycyclin, Tetracyclin und Chlortetracyclin gebildet wird.

3. Zusammensetzung nach Anspruch 2,
**dadurch gekennzeichnet,**
daß das Tetracyclin Doxycylin ist.

4. Zusammensetzung nach einem der Ansprüche 1 bis 3,
**dadurch gekennzeichnet,**
daß die Zusammensetzung Hydrochlorsäure enthält.

5. Zusammensetzung nach einem der Ansprüche 1 bis 4,
**dadurch gekennzeichnet,**
daß die Zusammensetzung Natriumformaldehydsulfoxylat enthält, das die Rolle eines Konseivierungsmittels spielt.

6. Zusammensetzung nach einem der Ansprüche 1 bis 5,
**dadurch gekennzeichnet,**
daß in der Zusammensetzung der Gewichtsanteil des Doxycyclin zwischen 5 % und 40 % in Form von Chlorhydraten mit einer Aktivität von mindestens 822 UI/mg beträgt.

7. Zusammensetzung nach einem der Ansprüche 1 bis 6,
**dadurch gekennzeichnet,**
daß in der Zusammensetzung der Gewichtsanteil des N(Hydroxyethyl)-2-Pyrrolidonzwischen 5 % und 80 % beträgt.

8. Zusammensetzung nach einem der Ansprüche 1 bis 7,
**dadurch gekennzeichnet,**
daß die Zusammensetzung zu Injektionszwekken verwendet wird.

9. Verfahren zur Herstellung einer Zusammensetzung gemäß einem der Ansprüche 1 bis 8,
**dadurch gekennzeichnet,**
daß zwei Drittel Gewichtsanteile des N-(Hydroxyethyl)-2-Pyrrolidon mit einem Drittel destilliertem Wasser gemischt werden, daß die Temperatur auf 50 C° heraufgesetzt wird, daß anschließend unter Rühren eine Mischung hauptsächlich Magnesiumoxid, dann fortschreitend Tetracycling, insbesondere DoxycyclinHydrochlorid hinzugefügt wird, daß der pH-Wert auf einen Wert etwa zwischen 5 und 7 gebracht wird, indem konzentrierte Hydrochlorsäure hinzugefügt und mit destilliertem Wasser aufgefüllt wird.